# EUROPEAN PATENT APPLICATION

(11) **EP 4 813 916 A1**
(43) Date of publication of application: **30.09.2026**
(21) Application number: 24894159.3
(22) Date of filing: 20.11.2024
(51) Int. Cl.: C01B 33/40, A23L 29/00, A61K 8/25, A61K 9/20, A61K 47/02, A61Q 1/00, A61Q 1/10, A61Q 1/12, A61Q 1/14, A61Q 19/00, A61Q 19/10

(54) **SILICATE MINERAL AND METHOD FOR PRODUCING SAME**

(30) Priority: 24.11.2023 JP 2023198887; 28.06.2024 JP 2024104560
(71) Applicant: Super Nano Design Co.,Ltd, Sendai-shi Miyagi 980-8579 (JP)
(72) Inventor: AJIRI, Tadafumi, Sendai-shi, Miyagi 980-8579 (JP); OHARA, Satoshi, Sendai-shi, Miyagi 980-8579 (JP); NAKANISHI, Ryo, Sendai-shi, Miyagi 980-8579 (JP)
(74) Representative: Hoffmann Eitle
(86) International application number: PCT/JP2024/041068
(87) International publication number: WO 2025/110168

(57) **Abstract**

[Problem] To make it possible to provide a silicate mineral having an average particle diameter larger than several hundred nanometers without containing impurities such as crystalline silica.

[Solution] The silicate mineral of the present invention has contents of each of crystalline silica and asbestos of 0.1 wt% or less. It is preferable that the mineral contains a carbonate, and it is preferable that it has an average particle diameter of 100 nm or more. Further, the mineral is suitably used in cosmetics, sanitary products, pharmaceuticals, and foods. The silicate mineral of the present invention is obtained by subjecting a natural silicate mineral to warm-water or hot-water treatment, or hydrothermal reaction treatment, at pH 9.4 or less.

## Description

### [Technical Field]

The present invention relates to a silicate mineral and a method for producing the same.

### [Background Art]

Silicate compounds are very diverse among minerals, and are widely used in cosmetics, foods, pharmaceuticals, and industrial products. Deep underground, groundwater is heated by volcanic activity to become high-temperature, high-pressure hot water (subcritical and supercritical water), dissolves rocks and becomes a supercritical aqueous solution state, and, when depressurized and cooled near the surface, their solubility decreases and they precipitate. This is the principle by which ore veins are formed. Silicate compounds are observed as main constituent components also in surface soil and sand. Silicate compounds, not only silica SiO₂ but also many minerals containing various metals such as Al, Ca, Fe, K, Na, Mg, exemplified by calcium silicate, magnesium silicate, iron silicate, sodium silicate, and the like, or minerals containing multiple metals, and many hydrates thereof are also formed.

**[Table 1]**

| Classification name | Structure | Chemical formula | Mineral examples |
|---|---|---|---|
| Nesosilicate minerals | Single tetrahedron | [SiO₄]⁴⁻ | Olivine, garnet, zircon, etc. |
| Sorosilicate minerals | Double tetrahedron | [Si₂O₇]⁶⁻ | Epidote, melilite group, etc. |
| Cyclosilicate minerals | Ring | [SiₙO₃ₙ]²ⁿ⁻ | Beryl group, tourmaline group, etc. |
| Single-chain inosilicate minerals | Single chain | [SiₙO₃ₙ]²ⁿ⁻ | Pyroxene group, etc. |
| Double-chain inosilicate minerals | Double chain | [Si₄ₙO₁₁ₙ]⁶ⁿ⁻ | Amphibole group, etc. |
| Phyllosilicate minerals | Layered | [Si₂ₙO₅ₙ]²ⁿ⁻ | Mica group, clay minerals, etc. |
| Tectosilicate minerals | Three-dimensional framework | [AlₓSi_{y}O_{(2x+2y)}]^{x-} | Quartz, feldspar, zeolite, etc. |

Given the nature of precipitation, impurities are often naturally contained. Among them, SiO₂, which is a constituent component, is also often contained as an impurity.

The present invention is intended to remove these impurities generated in the process of precipitation of a silicate mineral, and targets aluminum silicate, calcium silicate, magnesium silicate, iron silicate, etc. and hydrates thereof. Hereinafter, the present technology will be described taking hydrous magnesium silicate (also referred to as talcum, talc, etc.) as an example, but this is a technology that can be applied, in principle, to other silicate minerals as well.

Hydrous magnesium silicate is widely used in cosmetics, foods, pharmaceuticals, and industrial products, but since it is a natural mineral, it often contains impurities. In commercial use, toxic substances, heavy metals, etc. are separated, removed, and used. However, in many cases, these materials including talc which is a natural mineral tend to be mixed with components such as SiO₂ that tend to co-precipitate at the time of mineral formation, and these components that tend to co-precipitate are often mixed into products. For example, they are often found to contain small amounts of tremolite and chrysotile due to asbestos. Contamination with asbestos is not permitted in cosmetics, foods, pharmaceuticals, etc., of course, and is also not permitted in industrial products, and contamination with other crystalline SiO₂ (also referred to as crystalline silica, quartz, etc.) has also recently come to be regarded as a problem.

However, since mineral formation underground occurs via precipitation driven by temperature and pressure, the composition of precipitates differs depending on the place where the mineral is mined. It has been considered that the only solution to the problem is to closely examine, select, and use a portion containing almost no impurities as described above, but even then impurities are often contained in trace amounts, and this is a major problem particularly for applications in fields such as medical care, foods, cosmetics, etc.

Further, focusing on crystalline SiO₂, since the physical properties required for the separation of existing components of hydrous magnesium silicate and SiO₂, such as specific gravity, are almost the same, it has been considered that separation thereof is impossible not only by centrifugation, sedimentation, and gravity separation, but also by adsorption operations such as chromatography.

Here, it has been proposed to artificially synthesize talc in the form of fine particles by using hydrothermal synthesis including a supercritical field (see Patent Literature 1).

### [Citation List]

### [Patent Literature]

[Patent Literature 1] JP2014-520743A

### [Summary of the Invention]

### [Problems to be Solved by the Invention]

However, synthetic talc obtained by the method described in Patent Literature 1 has a small particle diameter of 20 nm to 100 nm. In application fields where hydrous silicate compounds such as talc are actually used, since natural minerals are used by sieving after pulverization, the particle diameter is several tens of micrometers or more, or is sub-micrometers or more even in small cases, and from the perspective of user safety (nano risk), it is desirable to provide talc having a larger particle diameter. In principle, an increase in particle diameter is possible by a hydrothermal method or the like, but artificial synthesis has issues in manufacturing cost, productivity, and optimization of properties, and in practice has not become a method for industrially producing particles of several hundred nm or more.

Therefore, even if one tries to remove impurities from a natural mineral, from the perspective of crystalline silica, in the sense of dissolving and removing SiO₂ impurities from a mixed system of hydrous magnesium silicate and SiO₂ impurities, it is possible to dissolve the impurities by setting alkaline conditions, but hydrous magnesium silicate is also dissolved at the same time. Therefore, separation and removal studies for leaving hydrous magnesium silicate while dissolving and removing SiO₂ impurities have not yet been conducted. Ideally, for a method for industrial applications, conditions are required under which only SiO₂ is dissolved without dissolving hydrous magnesium silicate. However, in view of the fact that, in nature, multiple minerals form in parallel, they are mixed because the solubilities are almost the same, and searching for such conditions is extremely difficult.

Further, regarding asbestos, asbestos is a serpentine mineral and an amphibole mineral, and is generally resistant not only to heat but also to both acids and alkalis. That is, in conditions under which the removal by dissolution may be achieved, other components, for example, hydrous magnesium silicate itself, would also be dissolved, and the removal is generally very difficult.

The present invention has been made in view of such problems, and is to make it possible to provide a silicate mineral having an average particle diameter larger than several hundred nm without containing impurities such as crystalline silica.

### [Means for Solving the Problems]

As a result of intensive studies, the present inventors have found that, by subjecting a silicate mineral derived from a natural mineral to warm-water or hot-water treatment, or hydrothermal reaction treatment, at pH 9.4 or less, toxic impurities such as crystalline silica and asbestos can be dissolved in water, or can be removed by reaction modification, and have completed the present invention. Specifically, the present invention provides the following:

The present invention is a method for producing a silicate mineral powder, comprising a step of subjecting a silicate mineral derived from a natural mineral to warm-water or hot-water treatment, or hydrothermal reaction treatment, at pH 9.4 or less. Further, carbonic acid or the like can also be allowed to coexist in the reaction field, whereby pH is controlled to be low and reaction modification removal of impurities is also enabled.

Hydrothermal reactions are widely used as a single-crystal growth method for metal oxide crystals, including quartz. Under hydrothermal conditions, metal oxides repeatedly dissolve and precipitate and undergo crystal growth. This crystal growth is called Ostwald ripening. Unstable fine particles and crystal tips having high surface energy are more likely to dissolve, and single-crystal growth proceeds so that more stable surfaces are exposed. In practice, this mechanism and principle of Ostwald ripening are used industrially as a method for promoting faster crystal growth and producing large single crystals.

Since the solubility of a metal oxide differs depending on the temperature, in general, in order to proceed with this single-crystal growth, crystal growth is performed at higher speed by creating a temperature distribution in a crystal growth apparatus. Under subcritical hydrothermal conditions and high-pressure supercritical conditions (high water density), a method is employed in which a raw material is dissolved in a high-temperature field and seed crystals are grown in a low-temperature field. When using a supercritical water state of relatively low pressure and low density, precipitation instead occurs in a high-temperature (low water density) field, and the natural retention of precipitates is positively utilized. Such precipitation usually requires at least several hours, and generally requires several days to several weeks.

Considering the principle of this Ostwald ripening, if hydrous magnesium silicate such as talc having various particle diameters and shapes is hydrothermally ripened, dissolution occurs from fine particles and tip-shaped particles in all components. The present invention differs from the crystal growth method, which aims at the growth of tremolite, chrysotile, and crystalline silica (quartz) that can be contained as impurities, and is instead a method for the removal of impurities by dissolution or reaction modification, and produces a silicate-based mineral free of impurities such as crystalline silica by causing dissolution or reaction modification of impurities such as crystalline silica more selectively while suppressing dissolution of the silicate mineral.

Further, compared with artificial synthesis of talc using hydrothermal synthesis, which is, in principle, similarly capable of producing a silicate mineral that does not contain crystalline silica or chrysotile as an impurity, the present invention is superior in terms of manufacturing cost. Further, since the raw material is a pulverized product of a natural mineral, it is possible to recover particles having a particle diameter of several hundred nanometers or more as demanded on the market.

Since the reaction field is one in which Ostwald ripening occurs, morphologically the recovered silicate mineral particles include fewer fine-particle products and have a rounded shape compared with general pulverized natural products. Further, hydroxyl groups on the surface of the product are more abundant.

The pH is 9.4 or less. Under high pH, crystalline silica can be dissolved, but dissolution of silicate minerals occurs at the same time. Fundamentally, for subcritical and supercritical hydrothermal synthesis (artificial synthesis) of a silicate mineral, low pH is desirable. This condition allows a silicate mineral to precipitate, so that it is considered to be desirable for dissolving only crystalline silica without dissolving the silicate mineral, in principle.

The same idea can be applied to the removal of asbestos-type impurities such as chrysotile. Since asbestos such as chrysotile is a mineral exhibiting basicity, by not setting the pH to be high under hydrothermal conditions, it becomes, in principle, possible to set conditions for dissolving these and precipitating the silicate mineral.

These impurities become particularly problematic when they are needle-like products, and it is under hydrothermal conditions that a needle-like substance dissolves from its tip. Considering the dissolution rate and dissolution amount of the large quantity of silicate mineral present, even if silicate minerals are dissolved in the same quantity as the impurities, it is ultimately possible to leave only the silicate mineral present in a large amount.

An acid can be allowed to coexist to lower the pH, and carbonic acid can be used as the acid. In this case, depending on the conditions, carbonate formation may occur. As a mechanism of talc formation in a reaction field of mineral formation occurring deep underground, the following reaction is known.

[Equation 1] . 2 Mg₃Si₂O₅(OH)₄ + 3CO₂ → Mg₃Si₄O₁₀(OH)₂ + 3MgCO₃ + 3H₂O

This indicates that serpentine (chrysotile) reacts with CO₂ under hydrothermal conditions to produce talc. The magnesium of chrysotile precipitates as magnesium carbonate. Since its solubility under hydrothermal conditions is higher than that of other products, the magnesium carbonate can also be removed by dissolution via a semi-batch extraction operation.

Further, in recent studies, carbonation by a reaction between calcium silicate or the like and CO₂ has been reported. Not only research on mineral formation mechanisms but also hardening by carbonation of calcium silicate compounds (Goto et al., Inorganic Materials, Vol. 5, Jan. 22-27, 1998), absorption of CO₂ into concrete (CO₂-SUICOM [registered trademark] process), and artificial marble synthesis by a hydrothermal reaction with CO₂ coexisting with calcium silicate by Professor Richard Riman of Rutgers University in the United States have been put into practical use as research and development and technological development for solving the CO₂ problem. That is, under hydrothermal conditions in the presence of CO₂, carbonation proceeds.

On the other hand, in the field of geophysics, the following is known as another mechanism of talc formation underground.

[Equation 2] **3 CaMg(CO₃)₂ + 4 SiO₂ + H₂O → Mg₃Si₄O₁₀(OH)₂ + 3 CaCO₃ + 3 CO₂**

This reaction mechanism is a reaction in which talc is generated with silica coexisting under hydrothermal conditions. It suggests that it is possible to modify to talc via reaction between carbonate and silica as described above.

That is, tremolite mixed as a trace component is:

Ca₂(Mg,Fe)₅Si₈O₂₂(OH)₂

(where Mg/(Mg+Fe)=1.0-0.9)
however, when tremolite is mixed in a trace amount, under hydrothermal conditions, not only removal by dissolution but also, in the presence of CO₂, carbonation occurs, and, at the same time, silica components present as impurities similarly react while dissolving, whereby it is modified into talc.

Further, in the present invention, it is preferable that the warm-water or hot-water treatment, or the hydrothermal reaction treatment, is performed in the presence of Mg ions. When crystalline silica or the like is contained in a mineral, it is also possible, in terms of equilibrium, that generation of magnesium silicate is promoted by allowing Mg to coexist. Simply, there is also a reaction in which SiO₂ complexes with Mg. However, in practice, Mg ions and Si ions coexist due to dissolution of silica and magnesium silicate, but if magnesium ions are supplied, it is also possible to cause dissolution equilibrium of the silica and magnesium silicate to preferentially cause dissolution of the silica.

The silicate mineral derived from natural minerals as a raw material is obtained by pulverizing them. Therefore, it is difficult to make the average particle diameter less than 200 nm and, also for the product after hydrothermal reaction treatment, the average particle diameter becomes 200 nm or more.

Therefore, according to the present invention, talc having an average particle diameter larger than 100 nm can be provided without containing impurities such as crystalline silica and asbestos components.

Further, in the present invention, it is preferable that the temperature in the warm-water or hot-water treatment, or the hydrothermal reaction treatment ranges from 70°C to 370°C, and the pressure is not less than the saturated vapor pressure of water.

According to the present invention, since impurities such as crystalline silica or asbestos can be dissolved and removed by a reaction using low-temperature heat of 370°C or less (preferably 300°C or less, more preferably 250°C or less, even more preferably 200°C or less, particularly preferably 150°C or less), it is superior in terms of the manufacturing cost. Not only heat from a heat source device but also waste heat recovered in a factory and the like can be considered as the low-temperature heat.

The present invention can be applied to any of a batch apparatus, a semi-batch apparatus, and a flow-through apparatus, but it is preferable to perform warm-water or hot-water treatment, or hydrothermal reaction treatment, using a semi-batch apparatus or a flow-through apparatus, and it is more preferable to perform warm-water or hot-water treatment, or hydrothermal reaction treatment, using a semi-batch apparatus.

The solubility of silica in pure water has been reported. However, in the case of hydrothermal treatment of a mineral containing other ions as in this system, since dissolution of those minerals occurs naturally, the solubility here differs from that of silica in high-temperature, high-pressure water. In general, solubility and dissolved chemical species concentration of a specific component in the presence of other minerals can be solved by simultaneously solving dissolution equilibrium equations of all substances, and further dissociation equilibrium of water and charge balance. The necessary chemical equilibrium can be predicted with high accuracy including the supercritical region by the Helgeson Kirkham Flowers (HKF) model or an improved HKF model by Sue et al. (Sue, K., Hakuta, Y., Smith, R. L., Adschiri, T., & Arai, K. (1999). Solubility of lead(II) oxide and copper(II) oxide in subcritical and supercritical water. Journal of Chemical & Engineering Data, 44(6), 1422-1426. https://doi.org/10.1021/je9901029)

For example, the saturated solubility becomes greater due to the addition of an alkali or the influence of coexisting ions. Further, in a mineral precipitation process underground, when a mineral precipitates and when silica precipitates as an impurity, it is suggested that the solubility of the precipitated mineral is low and the precipitation rate is high. Not only from the above equilibrium theory but also kinetically, it is inferred that silica is in a situation where it tends to dissolve. This has been verified experimentally, and the results can sufficiently explain that dissolution that is ten times or more faster is achieved not only in terms of equilibrium theory but also kinetically.

For an operating reaction system, the optimum value of a water flow rate at the time of operation is determined from this equilibrium theory and kinetics.

By using a semi-batch apparatus, heat recovery and preheating in warm-water and hot-water treatment or hydrothermal reaction treatment can also be performed, and thus it is even more superior in terms of manufacturing cost. Further, since impurities such as crystalline silica and asbestos that have been extracted and removed can be excluded from the system, contamination due to reprecipitation thereof can be prevented.

When the reaction apparatus is a semi-batch apparatus, the amount of an aqueous solvent supplied to the semi-batch apparatus is preferably 0.1 times or more of the theoretical amount required to saturate and dissolve crystalline silica contained in the silicate mineral as a raw material in a reaction solution containing coexisting ions in a reaction field. Alternatively, the amount of crystalline silica contained in the silicate mineral as a raw material introduced into the semi-batch apparatus is preferably 10 times or less of the theoretical amount required to saturate and dissolve the crystalline silica in a reaction solution containing coexisting ions in a reaction field.

When a flow-through apparatus is used, a silicate mineral is supplied in a suspended state in water, and warm-water or hot-water treatment or hydrothermal reaction treatment is performed. Recovery of heat at the outlet and its further use for preheating of the raw material can be performed, and unlike a semi-batch apparatus, since heat loss upon a temperature rise and cooling of the tank as the extraction means is eliminated, the heat recovery rate is improved. Regarding the cooling portion, sufficiently dissolving crystalline silica and rapidly cooling can suppress reprecipitation of the dissolved silica or the like in a downstream cooling portion through inhibition of Ostwald ripening and recrystallization growth of residual silica therein. Note that studies of these conditions can be performed by a small batch test.

Further, the concentration of a silicate-mineral aqueous slurry supplied to the flow-through apparatus is preferably 0.1 times or more of the theoretical amount required to saturate and dissolve crystalline silica contained in the silicate mineral as a raw material in a reaction solution containing coexisting ions in a reaction field.

### [Effect of the Invention]

According to the present invention, a silicate mineral having an average particle diameter larger than several hundred nanometers can be provided without containing impurities such as crystalline silica.

### Brief Description of Drawings

[FIG. 1] FIG. 1 is a schematic diagram of manufacturing apparatus 1 of the present embodiment.
[FIG. 2] FIG. 2 shows an X-ray diffraction test result of natural talc before hydrothermal reaction treatment.
[FIG. 3] FIG. 3 shows an X-ray diffraction test result of natural talc after hydrothermal reaction treatment.

### Modes for Carrying Out the Invention

Hereinafter, specific embodiments of the present invention will be described in detail, but the present invention is not limited to the following embodiments and can be implemented with appropriate modifications within the scope of the objectives of the present invention.

### <Manufacturing apparatus for hydrous magnesium silicate powder>

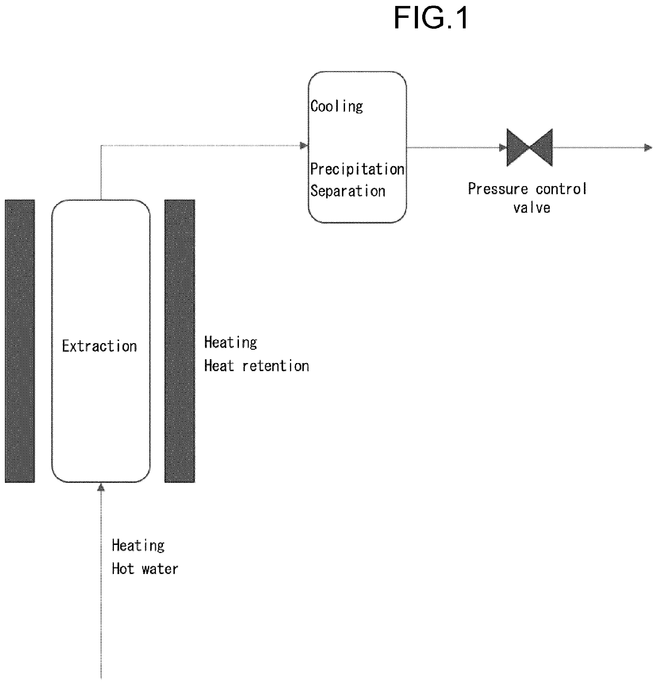

FIG. 1 is a schematic diagram of manufacturing apparatus 1 for hydrous magnesium silicate powder.

Manufacturing apparatus 1 includes extraction means 10 and, as necessary, cooling means 20.

### {Extraction means 10}

Extraction means 10 is an apparatus that brings a raw material liquid and an aqueous material into contact and subjects the raw material to warm-water or hot-water treatment, or hydrothermal reaction treatment. Extraction means 10 may be in the form of any of a batch apparatus, a semi-batch apparatus, and a continuous apparatus, but, in that the temperature in hydrothermal reaction treatment or the like can be made lower and not only heat from a heat source device but also waste heat in a factory can be used, thereby improving the manufacturing cost, extraction means 10 is preferably a batch apparatus or a semi-batch apparatus, and it is more preferable to perform warm-water or hot-water treatment, or hydrothermal reaction treatment, using a semi-batch apparatus.

By using a semi-batch apparatus, heat recovery and preheating in warm-water and hot-water treatment or hydrothermal reaction treatment can also be performed, and thus it is even more superior in terms of manufacturing cost. Further, since impurities such as crystalline silica and asbestos that have been extracted and removed can be excluded from the system, contamination due to reprecipitation thereof can be prevented.

Further, extraction means 10 may be in the form of a continuous apparatus (flow-through apparatus). When a flow-through apparatus is used, a silicate mineral is supplied in a suspended state in water, and warm-water or hot-water treatment or hydrothermal reaction treatment is performed. Recovery of heat at the outlet and its further use for preheating of the raw material can be performed, and unlike a semi-batch apparatus, since heat loss upon temperature rise and cooling of a tank as the extraction means is eliminated, the heat recovery rate is improved. Regarding the cooling portion, sufficiently dissolving crystalline silica and rapidly cooling can suppress reprecipitation of the dissolved silica or the like in a downstream cooling portion through inhibition of Ostwald ripening and recrystallization growth of residual silica therein. Note that studies of these conditions can be performed by a small batch test.

Hereinafter, unless otherwise specified, descriptions will be presented assuming that extraction means 10 is a semi-batch apparatus, but the present invention is not limited thereto.

### [Raw Materials]

The raw material introduced into extraction means 10 is a silicate mineral, and may be derived from a natural mineral or may be derived from a synthetic mineral, but is preferably a silicate mineral derived from a natural mineral.

The type of metal constituting the silicate mineral is not particularly limited, and includes alkali metals and alkaline earth metals, as well as aluminum, iron, etc. Specific examples of the silicate mineral include aluminum silicate, magnesium silicate, calcium silicate, iron silicate, or a silicate mineral containing multiple kinds of those metals, etc. Further, the silicate mineral may be a hydrous silicate mineral as a hydrate thereof.

For example, when the silicate mineral is a natural mineral including talc (hydrous magnesium silicate), according to the Order for Enforcement of the Industrial Safety and Health Act (Japanese Cabinet Order No. 318 of August 19, 1972) and the Ordinance on Prevention of Health Impairment due to Asbestos (Ordinance of the Japanese Ministry of Health, Labour and Welfare No. 21 of 2005), the content rate of asbestos (tremolite, chrysotile, etc.) contained in the natural mineral must be 0.1% or less. Therefore, it is preferable to use, as the raw material, a silicate mineral having a content rate of 0.1% or less.

Incidentally, as a method for determining the content rate of asbestos in talc using an X-ray diffraction method, "Method for analyzing asbestos content in natural minerals" which is an attachment to Kian-ka-hatsu No. 0828001 is adopted. This method uses a general-purpose X-ray diffraction apparatus (XRD apparatus), and the measurement conditions are as follows ("Method for analyzing asbestos content in talc," Asbestos Analysis Manual for Preliminary Survey under the Asbestos Regulation, March 2018, Ministry of Health, Labour and Welfare).
Tube voltage: 40 kV or more
Tube current: 30 mA or more
Anticathode: Cu
Monochromatization: graphite monochromator or Ni filter
Detector: scintillation counter, proportional counter, Geiger counter, semiconductor detector, etc.
Slit system: receiving slit 0.3 mm or 0.2 mm
Divergence slit: 1°
Scattering slit: 1°
Goniometer scanning speed: 1/8° per minute or less
Time constant: An appropriate time constant is used.
Full scale of chart: For measurement of diffraction line intensity, a net peak area is obtained by subtracting the background. An appropriate full scale is chosen such that the diffraction line appears as a peak on the recording chart.

In general, in measurement of a trace component, the measurement peak may be hidden by baseline noise, and in order to increase the signal-to-noise ratio, it is necessary to set a long integration time. Thereby, in principle, trace detection is possible even without using a powerful radiation source.

However, when evaluated within the measurement time used for general crystal structure analysis, the integration time is insufficient and the peak cannot be sufficiently detected, and it may also be possible that the asbestos content rate is determined to be 0.1 wt% or less which is the safety standard.

In the invention described in the present embodiment, analysis is performed from the result of performing a long-time measurement with sufficient consideration to this point and creating a calibration curve based on precise baseline evaluation. It has been confirmed that asbestos is removed by reaction modification and the asbestos content truly achieves 0.1 wt% or less, which is the safety standard specified in the attachment to Notification No. 0828001 issued by the Director, Chemical Substances Management Division, Industrial Safety and Health Department, Japanese Labour Standards Bureau, by means of a determination result of sufficient accuracy obtained by making full use of the precise analysis method prescribed in the said attachment, even when using a general-purpose X-ray diffraction apparatus.

### [Dispersion medium]

Further, the dispersion medium for dispersing the raw material is an aqueous material. The aqueous material refers to water, a polar organic solvent, or a mixed solvent of water and a polar organic solvent. As the aqueous material, for example, water, alcohols, carboxylic acids, ketones, ethers, esters, amides, amines, sulfur compounds, etc., and mixtures thereof, etc. can be mentioned.

Examples of the alcohols include methanol, ethanol, isopropyl alcohol, t-butyl alcohol, propylene glycol, phenol, etc.

Examples of the carboxylic acids include lower carboxylic acids such as formic acid, acetic acid, propionic acid, butyric acid, valeric acid, caproic acid, etc.

Examples of the ketones include acetone, methyl ethyl ketone, methyl isobutyl ketone, etc.

Examples of the ethers include ethylene glycol monobutyl ether, ethylene glycol monoethyl ether, tetrahydrofuran, dioxane, methyl cellosolve, etc.

Examples of the esters include ethyl acetate, butyl acetate, etc.

Examples of the amides include formamide, dimethylformamide, acetamide, dimethylacetamide, nitromethane, acetonitrile, etc.

Examples of the amines include methylamine, ethylamine, trimethylamine, triethylamine, monoethanolamine, diethanolamine, triethanolamine, pyridine, ethylenediamine, hexamethylenediamine, etc.

Examples of the sulfur compounds include dimethyl sulfoxide, etc.

Among them, since handling is easy, the aqueous material preferably contains at least one selected from water, alcohols, and carboxylic acids, and is more preferably water.

Further, a pH adjuster and an oxidizing agent/reducing agent can also be added to the aqueous material for control of the reaction field.

Examples of the pH adjuster include, as an acid, hydrochloric acid, nitric acid, acetic acid, sulfuric acid, carbonic acid, or ammonium salts thereof, and as an alkali, potassium hydroxide, sodium hydroxide, calcium hydroxide, magnesium hydroxide, aluminum hydroxide, ammonia, etc.

Examples of the oxidizing agent/reducing agent include hydrogen peroxide, oxygen, nitric acid, and also formic acid, hydrazine, hydrogen, ammonia, ethanol, formaldehyde, etc.

These substances are not only simply related to the solubility of the target impurities but also function as reactive substances.

For example, by allowing carbonic acid or CO₂ to coexist in the aqueous material, carbonate formation occurs, and further, via reaction with silica, modification to a non-toxic carbonate mineral or to the formation of talc from a toxic impurity mineral may occur in some cases.

Further, it is preferable that Mg ions are added to the aqueous material. Thereby, warm-water or hot-water treatment, or hydrothermal reaction treatment, performed thereafter is to be performed in the presence of Mg ions.

When crystalline silica or the like is contained in a mineral, it is also possible, in terms of equilibrium, that generation of magnesium silicate is promoted by allowing Mg to coexist. Simply, there is also a reaction in which SiO₂ complexes with Mg. However, in practice, Mg ions and Si ions coexist due to dissolution of silica and magnesium silicate, but if magnesium ions are supplied, it is also possible to cause dissolution equilibrium of the silica and magnesium silicate to preferentially cause dissolution of silica. Therefore, it is preferable that Mg ions are added to the aqueous material.

### [Raw material liquid]

In the present embodiment, a raw material liquid in which the raw material is dispersed using the dispersion medium is introduced into extraction means 10. In terms of the mode, the raw material liquid can be introduced as powder or as a flowable fluid. This is not particularly limited as long as it has fluidity, and examples include an aqueous solution containing a raw material component, a slurry, a paste, a suspension, etc.

Note that, when it is difficult to prepare an aqueous slurry, the raw material may be dispersed in an aqueous material such as ethanol to form a slurry.

When the reaction apparatus is a semi-batch apparatus, the amount of aqueous solvent supplied to the semi-batch apparatus is preferably 0.1 times or more, more preferably 0.3 times or more, even more preferably 0.5 times or more, and still more preferably 0.8 times or more, of the theoretical amount required to saturate and dissolve crystalline silica contained in the silicate mineral as a raw material in a reaction solution containing coexisting ions in a reaction field. Alternatively, the amount of crystalline silica contained in the silicate mineral as a raw material introduce into the semi-batch apparatus is preferably 10 times or less, more preferably 3.5 times or less, even more preferably 2 times or less, and still more preferably 1.3 times or less, of the theoretical amount required to saturate and dissolve the crystalline silica in a reaction solution containing coexisting ions in a reaction field.

In the present specification, it is considered preferable that the amount of crystalline silica is 0.1 times or more of the theoretical amount to be saturated and dissolved in a reaction solution containing coexisting ions in a reaction field. The amount of crystalline silica is preferably equal to the theoretical amount of water to be saturated and dissolved or more, and there is little flexibility, in terms of simple chemical equilibrium theory. However, in the present invention, reaction kinetics rather than chemical equilibrium theory is dominant, and the present invention utilizes the property that silica is more easily dissolved than talc. For example, when an alkali is added, or when Mg ions are added, chemical equilibrium changes. Further, since the dissolution rate of crystalline silica becomes higher than that of other minerals such as talc at the same time, in processing in a semi-batch apparatus or a flow-through apparatus, or in batch processing in a short time, it becomes kinetically advantageous, and thus the amount of crystalline silica can be smaller than the theoretical amount of water to be saturated and dissolved. Therefore, since there is a substantial flexibility in the amount of crystalline silica, it is sufficient if it is 0.1 times or more of the theoretical amount.

The same applies to a case where extraction means 10 is a flow-through apparatus, and the concentration of a silicate-mineral aqueous slurry supplied to the flow-through apparatus is preferably 0.1 times or more, more preferably 0.3 times or more, even more preferably 0.5 times or more, and still more preferably 0.8 times or more, of the theoretical amount required to saturate and dissolve crystalline silica contained in the silicate mineral as a raw material in a reaction solution containing coexisting ions in a reaction field.

Note that, in the present invention, the theoretical amount to be saturated and dissolved in the reaction solution containing coexisting ions in the reaction field can be obtained using the Helgeson Kirkham Flowers (HKF) model. As for the solubility evaluation method, it is as described above.

The pH of the raw material liquid introduced into extraction means 10 is 9.4 or less. If the pH exceeds 9.4, even if hydrothermal reaction treatment or the like is performed using hydrous magnesium silicate derived from a natural mineral as the raw material, there is a possibility that crystalline silica that can be present in the raw material cannot be sufficiently dissolved, and thus it is not preferable.

In order to more suitably dissolve impurities in the solvent, the upper limit of the pH is preferably 7 or less. Further, from the perspective of inducing modification from a mineral impurity to talc formation by allowing carbonic acid or CO₂ to coexist in the aqueous material, pH of 6 or less is more preferable, and of 5 or less is even more preferable.

The lower limit of the pH is not particularly limited, but from the perspective of suppressing the corrosion of apparatuses including extraction means 10, the lower limit of the pH is preferably 1 or more, and more preferably 2 or more. In addition, from the perspective of inducing modification from a mineral impurity to talc formation by allowing carbonic acid or CO₂ to coexist in the aqueous material, pH of 3 or more is more preferable, and of 4 or more is even more preferable.

The timing for allowing an acid or a base to coexist is not particularly limited, and it is sufficient if the acid or base coexists before the raw material liquid and the aqueous material come into contact in extraction means 10. However, to simplify the configuration of manufacturing apparatus 1, it is preferable to supply an acid or a base in the raw material preparation stage to make the raw material liquid in an acidic or basic state.

Although not essential, it is preferable that the raw material liquid is degassed. As a degassing apparatus for the raw material, for example, a degassing apparatus using ultrasonic waves, a degassing apparatus performing depressurization, a degassing apparatus that introduces a rare gas into the raw material liquid, a degassing apparatus using a permeation membrane, an existing degassing apparatus of existing technology, and a degassing apparatus combining these existing degassing apparatuses, etc. can be mentioned. By degassing the raw material liquid, corrosion of extraction means 10 and cooling means 20 due to dissolved oxygen can be suppressed.

### [Supply of aqueous material]

Next, an aqueous material continuously supplied to extraction means 10 will be described.

As the type of aqueous material, the materials described above as the dispersion medium can be mentioned.

Although not essential, it is preferable that the aqueous material is degassed. As a degassing apparatus for the aqueous material, the apparatus described above as the degassing apparatus for the raw material can be mentioned. By degassing the aqueous material, fluctuations in the amount of supplied aqueous material caused by bubbles or the like generated by dissolved gas are suppressed. Further, corrosion of extraction means 10 and cooling means 20 due to dissolved oxygen can be suppressed. Further, dissolved oxygen affects the redox state of a hydrothermal treatment reaction field, and the presence of gas that strongly affects a modification reaction under hydrothermal conditions such as CO₂ is also an important factor for controllable processing.

The aqueous material is in a pressurized state by the action of a pressurizing pump or the like. By pressurizing the aqueous material and further heating it to bring the aqueous material into a subcritical state, the aqueous material can be continuously supplied to extraction means 10.

It is preferable that the aqueous material after pressurization is hot water or an aqueous material in a subcritical state. When the aqueous material is water, subcritical water has high solubility for silica. Therefore, it is preferable that pressurized water in a liquid state (liquid phase) or a state including the liquid phase as a main phase is included. However, even water in a gaseous phase or water in a state referred to as water vapor (or steam) may, by capillary force, form a condensed phase between particles and exhibit effects similar to liquid water in some cases, and thus water in these states is also included. Further, when the aqueous material is in a supercritical state, high density is required in order to exhibit high solubility, and thus the pressure therefor needs to be not only equal to but higher than the critical pressure, and this is not desirable for industrial mass production. This is also because the amount of hydroxyl groups generated on the surface of the raw material is smaller than in a non-supercritical state, and thus it can affect the dissolution of crystalline silica and asbestos that can be contained in the raw material.

The "warm-water conditions, hot-water conditions, or hydrothermal conditions" according to the present invention are defined as conditions in which liquid water can coexist having a reaction temperature of 70°C or more and 370°C or less. However, when fine particles are a target, since there is also reaction and dissolution activity in a condensed state by capillary force between fine particles, in such a special case, conditions in which the supply system includes water in a gaseous phase referred to as water vapor (or steam), may also be used.

The pressure of the aqueous material after pressurization is not less than the saturated vapor pressure. If it is less than the saturated vapor pressure, even if the raw material liquid and the pressurized aqueous material are brought into contact, there is a possibility that impurities such as crystalline silica and asbestos that can be contained in the raw material cannot be sufficiently dissolved or removed by reaction modification, and thus it is not preferable.

However, as a special example, when the particle diameter is small and capillary force acts between particles, even if the pressure is less than the saturated vapor pressure, that is, even in water in a state referred to as water vapor (or steam), a similar dissolution effect may be exhibited.

Since the removal of impurities such as crystalline silica and asbestos that can be contained in the raw material can be more suitably promoted, it is desirable that the pressure of the aqueous material after pressurization is not less than the saturated vapor pressure at the processing temperature, and, for example, when the processing temperature is 120°C, it is 0.2 MPa or more, and when 170°C, it is 0.8 MPa or more. It becomes lower at low temperature and higher at high temperature, and at the critical point of 374°C, it is 22.1 MPa or more. It is preferable that the pressure of the aqueous material after pressurization is 0.2 MPa or more, more preferably 0.5 MPa or more, and even more preferably 1 MPa or more. Even at a lower temperature, for example 70°C, since the solubility decreases, the processing efficiency and processing speed decrease, but, in principle, a similar removal effect is expected. In that case, since the pressure is 0.03 MPa and is not more than atmospheric pressure, processing can be performed at normal pressure and warm-water treatment can be performed.

Further, the pressure of the aqueous material after pressurization is 40 MPa or less in the supercritical region, more preferably 20 MPa or less, and even more preferably 10 MPa or less. However, basically, if it is not less than the saturated vapor pressure, a liquid phase is formed, and even if the pressure is increased beyond that, the water density hardly changes, and thus an increase in solubility cannot be expected. Therefore, if it is about several atmospheres higher than the saturated vapor pressure, a sufficient dissolution effect can be expected. Conversely, if the pressure of the aqueous material is too high, the cost of increasing the pressure resistance of manufacturing apparatus 1 significantly increases, and deterioration of extraction means 10 also tends to occur, and thus it is not preferable.

The type of heating apparatus for heating the aqueous material is not particularly limited. As the heating apparatus, for example, a heating apparatus that irradiates the aqueous material with microwaves, a heating apparatus that heats the aqueous material by heat conduction from a heat-generating body such as a heater, etc., can be mentioned. By heating the pressurized aqueous material, the aqueous material can be brought into a subcritical state.

High-temperature steam can also be utilized. By using that steam in a heat exchanger, or in combination with the heating apparatus described above, pressurized water can also be produced. When clean heated steam containing no impurities and having a temperature higher than the processing temperature can be obtained, it is also possible for that steam to be directly introduced. By controlling pressure with a pressure control valve to transform the steam into a liquid phase, it can be used as heated water for extraction.

If the temperature is 70°C or more, extraction of crystalline silica contained in the raw material is possible. Note that the saturated vapor pressure of water at 70°C is about 0.03 MPa, the saturated vapor pressure of water at 100°C is about 0.1 MPa, the saturated vapor pressure of water at 120°C is about 0.2 MPa, and the saturated vapor pressure of water at 170°C is about 0.8 MPa.

The temperature of the aqueous material after heating is 70°C or more. If the temperature is less than 70°C, even if the raw material liquid and the pressurized aqueous material are brought into contact, there is a possibility that impurities such as crystalline silica and asbestos that can be contained in the raw material cannot be sufficiently dissolved or removed by reaction modification, and thus it is not preferable.

Since dissolution of impurities such as crystalline silica and asbestos that can be contained in the raw material or removal thereof by reaction modification can be more suitably promoted, it is preferable that the temperature of the aqueous material after heating is 100°C or more, more preferably 120°C or more, and even more preferably 150°C or more.

Further, the temperature of the aqueous material after heating is 370°C or less, preferably 300°C or less, more preferably 250°C or less, and even more preferably 200°C or less. If the temperature of the aqueous material is too high, the amount of hydroxyl groups on the surface of the raw material is rather reduced, and thus it is not preferable.

According to the present embodiment, since impurities such as crystalline silica or asbestos can be dissolved and removed by a reaction using low-temperature heat of 370°C or less (preferably 300°C or less, more preferably 250°C or less, even more preferably 200°C or less, particularly preferably 150°C or less), it is even more superior in manufacturing cost. For low-temperature heating, not only heat from a heat source device but also reuse of waste heat in a factory can be considered.

### [Contact between raw material liquid and aqueous material]

By introducing the raw material liquid into extraction means 10 and thereafter continuously supplying the aqueous material to extraction means 10, the raw material liquid and the aqueous material come into contact. The raw material liquid is instantaneously heated to a subcritical temperature by the amount of heat held by the aqueous material, and reaction between the raw material liquid and the aqueous material is initiated. This reaction initiates an extraction reaction into the aqueous material or a modification reaction with respect to impurities such as crystalline silica and asbestos that can be contained in the raw material.

The shape of extraction means 10 is not particularly limited as long as it maintains a subcritical state for extracting crystalline silica into the aqueous material for a predetermined time. Regarding the shape of extraction means 10, for example, a spiral tube wound multiple times inside a heating cylinder, a reactor covered with a constant-temperature layer such as a molten salt bath jacket, a fluidized sand bath, etc., can be mentioned.

By making the shape of extraction means 10 a spiral tube wound multiple times inside a heating cylinder or a reactor covered with a constant-temperature layer, it is possible to prevent temperature fluctuations and temperature non-uniformity of a mixture of the raw material liquid and the aqueous material due to heat conduction through the apparatus wall surface and to realize the precise temperature control required in impurity extraction in a subcritical state.

From the outlet of extraction means 10, a high-temperature, high-pressure fluid in which impurities such as crystalline silica and asbestos are dissolved flows out. Since the solubility, that is, the solution concentration, is low, and the temperature and pressure are relatively low, it is also possible to remove it as it is. After extraction, a high-temperature, high-pressure fluid containing hydrous magnesium silicate derived from a natural mineral from which impurities have been removed is discharged from extraction means 10.

From the perspective of appropriately removing impurities such as crystalline silica and asbestos, it is preferable that the time of the hydrothermal reaction treatment reaction in extraction means 10 is 1 minute or more, more preferably 5 minutes or more, and even more preferably 10 minutes or more.

On the other hand, from the perspective of production efficiency and suppressing deterioration of extraction means 10, and in order to reduce the effect of impurity crystal growth due to Ostwald ripening, it is preferable that the time of the hydrothermal reaction treatment reaction is 10 hours or less, more preferably 5 hours or less, and even more preferably 2 hours or less.

### {Cooling Means 20}

The provision of cooling means 20 is not essential. Cooling means 20 is an optional configuration that may be provided as necessary. When the temperature and pressure are relatively high, or when the eluted concentration is high, cooling before the pressure control valve may cause reprecipitation of dissolved impurities and damage to the pressure control valve. In such a case, by providing cooling means 20, it is possible to lower the load on the pressure control valve in the subsequent stage by reprecipitating and removing the dissolved and extracted impurity components.

Cooling is performed by mixing a high-temperature high-pressure fluid supplied from extraction means 10 and a low-temperature, high-pressure aqueous material pressurized by the action of a pressurizing pump or the like. By mixing the high-temperature, high-pressure fluid and the low-temperature, high-pressure fluid, it is possible to promptly remove heat corresponding to the latent heat of vaporization accompanying a change in the state of the fluid, and to perform safe and stable operation. Further, if the high-temperature, high-pressure fluid is cooled by this mixing until it becomes equal to or less than the critical temperature, the high-temperature, high-pressure fluid is rapidly cooled and a reaction to produce particles can be stopped almost instantaneously. Therefore, it is possible to ensure that the product particles have almost uniform particle diameters.

When operating at a low temperature, such a cooling method is sufficient, but when operating at a relatively high temperature, heat recovery may be required from the perspective of energy utilization. In that case, a cooling tube is inserted to perform indirect cooling, that is, heat exchange, and the recovered heat is used for preheating of the raw material or flowing water.

Regarding recovery of particles from extraction means 10, when powder is introduced into a container, the particles are recovered as they are . When recovered as a slurry, the product-particle-containing fluid is separated into product particles and fluid through a filter. The type of filter is not particularly limited, and includes, for example, an in-line filter. The filter can collect hydrous magnesium silicate derived from a natural mineral from which impurities have been removed.

In a semi-batch operation, impurities are removed by dissolution, and thus cooling operation of the extraction means 10 is not important. However, when this system is used for a batch operation, dissolved impurities precipitate on the silicate mineral as the product by cooling. In that case, if the cooling rate is slow, reprecipitation of crystalline components may occur, and thus cooling operation of the entire apparatus becomes important.

### <Silicate mineral>

It is preferable that the average particle diameter of the silicate mineral obtained as the product is 100 nm or more. When the raw material is a natural mineral, the silicate mineral is provided by pulverizing the natural mineral. Therefore, it is difficult to make the average particle diameter less than 100 nm, and also for the product after hydrothermal reaction treatment, the average particle diameter becomes 100 nm or more.

From the perspective of safety of the user of talc, the average particle diameter is more preferably 200 nm or more, still more preferably 500 nm or more, even more preferably 1 µm or more, and particularly preferably 5 µm or more.

In general, when using a natural talc mineral, pulverization processing is performed and the product is often classified by sieving. In that case, the particle diameter becomes even larger as several tens of micrometers or more. In the case of finer particles, a gas-phase classification method is used, but even in that case, generally the diameter is about several micrometers, and recovery of sub-micron particles is also possible as a special case.

In the present invention, the average particle diameter refers to a median diameter D₅₀ as determined by a centrifugal sedimentation method in accordance with JIS R1619.

Further, from the perspective of efficiency of industrial production, the lower limit of the processing concentration of the silicate mineral is preferably 1 wt% or more, and more preferably 3 wt% or more. The upper limit of the processing concentration of the silicate mineral is preferably 30 wt% or less, and more preferably 20 wt% or less. Among these, given that impurities can be appropriately processed even when the concentration of impurities (crystalline silica or asbestos) in the silicate mineral is relatively high, the upper limit of the processing concentration of the silicate mineral is further preferably 10 wt% or less, and particularly preferably 5 wt% or less.

Further, the content of crystalline silica in the silicate mineral obtained as the product is 0.1 wt% or less, more preferably 0.08 wt% or less, still more preferably 0.05 wt% or less, and even more preferably below the limit of detection.

Further, the content of asbestos in the silicate mineral powder obtained as the product is 0.1 wt% or less, more preferably 0.08 wt% or less, still more preferably 0.05 wt% or less, and even more preferably below the limit of detection.

In the present embodiment, the contents of crystalline silica and asbestos are obtained using an X-ray diffraction apparatus. In that case, the conditions of the X-ray diffraction apparatus are as follows.
Tube voltage: 45 kV
Tube current: 200 mA
Anticathode: Cu
Monochromatization: graphite monochromator
Detector: scintillation counter SC-70S
Slit system: receiving slit box 1 1.000 mm
   Receiving slit box 2 1.125 mm
   Incident slit box 1.000 mm
   Longitudinal restriction slit 15 mm
Goniometer scanning speed: 0.10° per minute
Full scale of chart: For measurement of diffraction line intensity, a net peak area is obtained by subtracting the background. An appropriate full scale is chosen such that the diffraction line appears as a peak on the recording chart.

For example, an X-ray diffraction apparatus such as SmartLab 9MTP (manufactured by Rigaku Corporation) can be set to these conditions.

Among crystalline silica, the content of quartz is obtained, in powder X-ray diffraction, from the peak intensity at a diffraction angle (2θ) of 26.6° using a calibration curve obtained using a standard quartz sample. Further, the content of cristobalite is obtained, in powder X-ray diffraction, from the peak intensity at a diffraction angle (2θ) of 22.0° using a calibration curve obtained using a standard cristobalite sample. Further, the content of tridymite is obtained, in powder X-ray diffraction, from the peak intensities at diffraction angles (2θ) of 20.5° and 21.6° using a calibration curve obtained using a standard tridymite sample.

Among asbestos types, the content of tremolite is obtained, in powder X-ray diffraction, from the peak intensity at a diffraction angle (2θ) of 10.4° using a calibration curve obtained using a standard tremolite sample. Further, the content of chrysotile is obtained, in powder X-ray diffraction, from the peak intensities at diffraction angles (2θ) of 12.1° and 24.3° using a calibration curve obtained using a standard chrysotile sample.

Analysis of other types is performed in accordance with the Asbestos Analysis Manual for Preliminary Survey under the Asbestos Regulation [Version 1.20], March 2018, Japanese Ministry of Health, Labour and Welfare, "8.4.3.1. Method for analyzing asbestos content in talc."

In general, in measurement of a trace component, the measurement peak may be hidden by baseline noise, and in order to increase the signal-to-noise ratio, it is necessary to set a long integration time. Thereby, in principle, trace detection becomes possible with a general XRD analysis apparatus even without using a powerful radiation source. In the invention described in the present embodiment, analysis is performed from the result of performing a long-time measurement with sufficient consideration to this point and creating a calibration curve based on precise baseline evaluation. Thereby, it has been confirmed that crystalline silica or asbestos or the like is dissolved and removed into the aqueous material and the asbestos content truly achieves 0.1 wt% or less, which is the safety standard specified in the attachment to Notification No. 0828001 issued by the Director, Chemical Substances Management Division, Industrial Safety and Health Department, Japanese Labour Standards Bureau, by means of a determination result of sufficient accuracy obtained by making full use of the precise analysis method prescribed in the said attachment, even when using a general-purpose X-ray diffraction apparatus.

Further, whether the product is a silicate mineral powder or not is determined from diffraction peaks in powder X-ray diffraction. For example, whether the product is a hydrous magnesium silicate powder (talc powder) or not is determined by whether it has diffraction peaks at diffraction angles (2θ) of 9.45°, 18.97°, and 28.62° in powder X-ray diffraction as determined by the precise analysis method described above.

Further, the amount of an aqueous NaOH solution (0.01 M) required in the following method (Sears method) is preferably 180 µL or more, and more preferably 200 µL or more.
(1) Disperse 0.1 g of the silicate mineral powder in 10 mL of water.
(2) After adding 2 g of NaCl, adjust the pH to 4 or less with dilute hydrochloric acid (0.12 M).
(3) Add the aqueous NaOH solution (0.01 M) to adjust the pH to 4, and then measure the amount of the aqueous NaOH solution (0.01 M) required to shift the pH from 4 to 9.

By hydrothermally treating natural talc, the amount of OH groups on the surface increases. Further, the amount of OH groups increases with an increase in hydrothermal temperature. Whether hydrothermal treatment is performed or not also affects wettability. By hydrothermally treating natural talc, the contact angle becomes smaller. This means that wettability has improved, that is, hydrophilicity has improved. Thereby, it can be said that, by hydrothermally treating a silicate mineral, affinity with a polar solvent for cosmetics is improved and blendability is improved.

When the silicate mineral obtained by the present invention is a hydrous magnesium silicate powder, the hydrous magnesium silicate powder can be applied to the field of plastics (as a filler [improvement of rigidity, heat resistance, dimensional stability], crystal nucleating agent), papermaking (as a filler, pitch control agent, coating agent), paint (as an extender pigment [adjustment of viscosity and gloss], powder coating), electronic material (as a laminated board, molded product, resist ink, adhesive), ceramics (as glaze of ceramics, honeycomb ceramics raw material), rubber (as a filler [improvement of heat resistance, reinforcing properties, etc.], release agent), cosmetics (for foundation, body powder, baby powder, eye shadow, lipstick), sanitary products (for baby powder, preventing babie's prickly heat/rash, etc.), pharmaceutical (as an excipient of tablets, lubricant, lubricant to be applied to medical rubber gloves), food (as a gum base, manufacturing aid [anti-sticking]), agriculture (as an anti-caking agent for fertilizer, pesticide carrier), etc. In particular, since it is highly safe, at a level equivalent to that of synthetic talc, the hydrous magnesium silicate powder obtained by the present invention is preferably applied in the field of cosmetics, sanitary products, pharmaceutical and/or the food.

For example, when applied in the field of cosmetics, a cosmetic composition may contain, in addition to the silicate mineral of the present invention, various components exemplified by a coloring agent, an extender pigment, a glittering agent, an oily component, a moisturizer, a surfactant, a thickener, a preservative, an ultraviolet scattering agent, an antioxidant, a chelating agent, etc., as necessary.

Examples of the coloring agent include inorganic pigments, organic pigments, dyes, natural pigments, etc., but the coloring agent is not limited thereto.

Examples of the extender pigment include inorganic powders such as silica, mica, synthetic fluorphlogopite, glass powder, barium sulfate, kaolin, bentonite, hectorite, zeolite, bismuth oxychloride, zirconium oxide, magnesium oxide, aluminum oxide, calcium sulfate, barium sulfate, magnesium sulfate, calcium carbonate, magnesium carbonate, and talc, etc., but the extender pigment is not limited thereto. Further, organic powders such as nylon, polyethylene, silicone elastomers such as (vinyl dimethicone/methicone silsesquioxane) crosspolymer, polymethyl methacrylate, lauroyl lysine, silk powder, cellulose powder, dispersants such as polyvalent metal salts of long-chain fatty acids, and various wax powders, etc., can be mentioned, but the extender pigment is not limited thereto.

Examples of the glittering agent include those obtained by coating surfaces of plate-like powders such as mica, synthetic fluorphlogopite, glass, silica, alumina, etc., with coloring agents such as titanium oxide, iron oxide, silicon oxide, iron blue, chromium oxide, tin oxide, chromium hydroxide, gold, silver, carmine, organic pigments such as Red No. 202 and Yellow No. 4, etc., and those obtained by cutting film bases such as polyethylene terephthalate/polymethyl methacrylate laminated powder, polyethylene terephthalate/aluminum vapor-deposited powder, polyethylene terephthalate/gold vapor-deposited laminated powder, etc., into arbitrary shapes, etc., but the glittering agent is not limited thereto.

Examples of the oily component include hydrocarbon oils, ester oils, waxes, higher alcohols, and animal and vegetable oils, etc. As hydrocarbon oils, squalane, dodecane, tetradecane, hexadecane, etc.; as ester oils, phytosteryl macadamiate, octyldodecyl myristate, tri(caprylic/capric) glyceride, stearyl stearate, methylheptyl isostearate, hexyl laurate, isoacyl laurate, cocoalkyl (caprylate/caprate), isocetyl myristate, isostearyl isostearate, etc.; as waxes, beeswax, Japan wax, carnauba wax, rice bran wax, sunflower seed wax, candelilla wax, spermaceti wax, montan wax, etc.; as higher alcohols (monohydric alcohol having six or more carbon atoms), cetyl alcohol, stearyl alcohol, isostearyl alcohol, lauryl alcohol, behenyl alcohol, etc.; and as animal and vegetable oils, avocado oil, linseed oil, almond oil, olive oil, cocoa butter, sesame oil, wheat germ oil, safflower oil, jojoba oil, phytosteryl macadamiate, shea butter, turtle oil, camellia oil, persic oil, castor oil, grapeseed oil, macadamia nut oil, coconut oil, rosehip oil, soybean oil, yolk oil, hydrogenated castor oil, hydrogenated coconut oil, hydrogenated cocoa butter, hydrogenated turtle oil, hydrogenated mink oil, beef tallow, mink oil, lanolin (wool fat), and oily components extracted from these components can be mentioned, but the oily component is not limited thereto.

Examples of the moisturizer include polyhydric alcohols such as glycerin, 1,3-butylene glycol, propylene glycol, polyethylene glycol, diglycerin trehalose; polymer compounds such as sodium hyaluronate, heparinoid, sodium chondroitin sulfate, collagen, elastin, keratin, chitin, chitosan; amino acids such as glycine, aspartic acid, arginine; natural moisturizing factors such as sodium lactate, urea, sodium pyrrolidone carboxylate; lipids such as ceramide, cholesterol, phospholipids; plant extracts such as chamomile extract, witch hazel extract, tea extract, perilla extract, etc., but the moisturizer is not limited thereto.

As the surfactant, anionic surfactants, cationic surfactants, nonionic surfactants, and amphoteric surfactants can be used. For example, one or two or more selected from polyoxyethylene alkyl ethers such as polyoxyethylene lauryl ether, polyoxyethylene cetyl ether, monocetyl glyceryl ether, polyoxyethylene stearyl ether, polyoxyethylene oleyl ether, etc.; polyoxyethylene derivatives such as polyoxyalkyl allyl ether, polyoxyethylene distyrenated phenyl ether, polyoxyethylene hydrogenated castor oil, polyoxyethylene lanolin, etc.; sorbitan fatty acid esters such as sorbitan monolaurate, sorbitan monooleate, sorbitan sesquioleate, sorbitan monostearate, etc.; polyoxyethylene sorbitan fatty acid esters such as polyoxyethylene sorbitan monolaurate, polyoxyethylene sorbitan monostearate, polyoxyethylene sorbitan monooleate, etc.; polyoxyethylene sorbitol fatty acid esters such as polyoxyethylene sorbit tetraoleate, etc.; polyethylene glycol fatty acid esters such as polyethylene glycol monolaurate, polyethylene glycol monooleate, etc.; alkyl glyceryl ethers such as isostearyl glyceryl ether, etc.; and glycerin fatty acid esters such as glyceryl monobehenate, etc., can be mentioned. Further, one or two or more selected from anionic surfactants such as fatty acid monocarboxylate salts, polyoxyethylene alkyl ether acetate salts, alkylsulfocarboxylate salts, α-olefin sulfonate salts, polyoxyethylene alkyl sulfates, alkyl phosphate salts, polyoxyethylene alkyl ether phosphate esters, stearoyl methyl taurine and salts thereof, etc.; and amphoteric surfactants such as fatty acid amidopropyl betaine, alkyl imidazolium betaine, alkyl dimethylaminoacetate betaine, alkyl dimethylsulfobetaine, alkyl dimethylamine oxide, alkyl hydroxysulfobetaine, etc., can be mentioned, but the surfactant is not limited thereto.

Examples of the thickener include guar gum, locust bean gum, carrageenan, xanthan gum, carboxymethyl cellulose, hydroxymethyl cellulose, hydroxyethyl cellulose, hydroxypropyl cellulose, hydroxypropyl methyl cellulose, hydrophobically modified hydroxypropyl methyl cellulose, polyvinyl alcohol, polyvinylpyrrolidone, carboxyvinyl polymer, acrylic acid alkyl methacrylate copolymer, polyethylene glycol, bentonite, (hydroxyethyl acrylate/acryloyldimethyl taurine sodium) copolymer, (acryloyldimethyl taurine ammonium/vinylpyrrolidone) copolymer, etc., but the thickener is not limited thereto.

Examples of the preservative include benzoic acid, sodium benzoate, dehydroacetic acid, sodium dehydroacetate, isobutyl parahydroxybenzoate, isopropyl parahydroxybenzoate, butyl parahydroxybenzoate, ethyl parahydroxybenzoate, propyl parahydroxybenzoate, benzyl parahydroxybenzoate, methyl parahydroxybenzoate, phenoxyethanol, chlorobutanol, chlorhexidine, salicylic acid, benzalkonium chloride, cetyltrimethylammonium bromide, acrinol, benzethonium chloride, cresol, gluconic acid and derivatives thereof, povidone-iodine, potassium iodide, iodine, isopropyl methylphenol, triclocarban, triclosan, photosensitizer No. 101, photosensitizer No. 201, paraben, phenoxyethanol, 1,2-pentanediol, alkyl diamino glycine hydrochloride, piroctone olamine, miconazole, etc., but the preservative is not limited thereto.

Examples of the ultraviolet absorber include para-aminobenzoic acid, glyceryl para-aminobenzoate, ethyl dihydroxypropyl para-aminobenzoate, octyl dimethyl para-aminobenzoate, amyl para-dimethylaminobenzoate, hexyl diethylamino hydroxybenzoyl benzoate, methyl anthranilate, homomenthyl salicylate, 2-ethylhexyl salicylate, triethanolamine salicylate, 2-ethylhexyl para-methoxycinnamate, glyceryl mono-2-ethylhexanoate dipara-methoxycinnamate, methyl 2,5-diisopropylcinnamate, bis(trimethylsiloxy)silyl isopentyl trimethoxycinnamate, isopropyl para-methoxycinnamate, a mixture of isopropyl para-methoxycinnamate and diisopropylcinnamate ester, 2-ethoxyethyl para-methoxycinnamate, a diethanolamine salt of para-methoxycinnamic acid, 4-isopropyl dibenzoylmethane, 4-tert-butyl-4□□-methoxydibenzoylmethane, 2,4,6-tris[4-(2-ethylhexyl oxycarbonyl)anilino]-1,3,5-triazine, 2,4-bis-[{4-(2-ethylhexyl oxy)-2-hydroxy}-phenyl]-6-(4-methoxyphenyl)-1,3,5-triazine, 2,4-dihydroxybenzophenone, 2,2□-dihydroxy-4-methoxybenzophenone, 2-hydroxy-4-methoxybenzophenone, 2,2□-dihydroxy-4,4[1-dimethoxybenzophenone, 2,2□4,4□-tetrahydroxybenzophenone, 4-(2-β-glucopyranosiloxy)propoxy-2-hydroxybenzophenone, 2-hydroxy-4-n-octyloxybenzophenone, 2-ethylhexyl dimethoxybenzylidene dioxoimidazolidine propionate, octocrylene, cinoxate, phenylbenzimidazole sulfonic acid, 1-(3,4-dimethoxyphenyl)-4,4-dimethyl-1,3-pentanedione, 3-(4-methylbenzylidene)camphor, methylene bis-benzotriazolyl tetramethylbutylphenol, etc., but the ultraviolet absorber is not limited thereto.

Examples of the ultraviolet scattering agent include titanium oxide, zinc oxide, cerium oxide, etc., but the ultraviolet scattering agent is not limited thereto.

Examples of the antioxidant include natural vitamin E, tocopherol, dibutylhydroxytoluene, butylhydroxyanisole, sorbic acid, sodium sulfite, ascorbic acid, erythorbic acid, L-cysteine hydrochloride, etc., but the antioxidant is not limited thereto.
As the pH adjuster, inorganic acids (hydrochloric acid, sulfuric acid, phosphoric acid, polyphosphoric acid, boric acid, etc.), organic acids (lactic acid, acetic acid, citric acid, sodium citrate, tartaric acid, malic acid, succinic acid, sodium succinate, oxalic acid, gluconic acid, fumaric acid, propionic acid, acetic acid, aspartic acid, ε-aminocaproic acid, glutamic acid, aminoethyl sulfonic acid, etc.), gluconolactone, ammonium acetate, inorganic bases (sodium bicarbonate, sodium carbonate, potassium hydroxide, sodium hydroxide, calcium hydroxide, magnesium hydroxide, etc.), organic bases (monoethanolamine, triethanolamine, diisopropanolamine, triisopropanolamine, lysine, etc.) can be mentioned, but the pH adjuster is not limited thereto.

Examples of the chelating agent include ethylenediaminetetraacetic acid (edetic acid), salts of ethylenediaminetetraacetic acid (sodium salt (edetate sodium: Japanese Pharmacopoeia, EDTA-2Na, etc.), potassium salt, etc.), phytic acid, gluconic acid, polyphosphoric acid, metaphosphoric acid, etc., but the chelating agent is not limited thereto.

The coloring agents and extender pigments described above can be used after surface treatment with a surface treatment agent as necessary. As the surface treatment agent, for example, fluorine compound treatment, silicone treatment, silicone resin treatment, pendant treatment, silane coupling agent treatment, titanium coupling agent treatment, oil agent treatment, metal soap treatment, N-acyl lysine treatment, polyethylene glycol treatment, PVA treatment, polyacrylic acid treatment, hyaluronic acid treatment, alginic acid treatment, inorganic compound treatment, urethane crosslinked polymer treatment, plasma treatment, mechanochemical treatment, etc., can be used to perform surface treatment in advance, but the surface treatment agent is not limited thereto. Among them, metal soap treatment and urethane crosslinked polymer treatment are preferable, and, as the metal soap treatment, aluminum dimyristate treatment, aluminum stearate treatment, aluminum distearate treatment, etc., as the urethane crosslinked polymer treatment, (HDI/trimethylol hexyl lactone) crosspolymer treatment can be suitably used.

Conventionally, the formation of minerals underground has been understood as precipitation governed by temperature and pressure conditions, with the composition of precipitates varying depending on the location where the minerals occur. Scrutinizing and selecting portions containing almost none of the aforementioned impurities has been regarded as the only solution. However, according to the present invention, even with a raw material containing impurities exceeding the specified concentration, it may be possible to make the impurity content less than the specified concentration, and in that regard, the present invention is also particularly advantageous for companies involved in processing and companies selling silicate minerals.

In the present invention, "an article ... including a silicate mineral" means both a case where the silicate mineral is included in a composition and a case where the silicate mineral adheres to an article. For example, "a pharmaceutical including a silicate mineral" means both a case where the silicate mineral powder is included in a pharmaceutical composition as an excipient or lubricant of a tablet and a case where the silicate mineral powder adheres to a medical rubber glove.

### [Examples]

The present invention is described in detail below with reference to Examples; however, the invention is not limited thereto.

**[Table 2]**

| | Sample conditions | | | Hydrothermal reaction treatment conditions | | |
|---|---|---|---|---|---|---|
| | Impurity | Silicate mineral concentration | pH | Temperature | Pressure | Treatment time |
| Example 1-1 | Quartz | 10 wt% | 9.4 | 150°C | 0.5 MPa | 10 min |
| Example 1-2 | Quartz | 10 wt% | 9.4 | 150°C | 0.5 MPa | 5 min |
| Example 1-3 | Quartz | 10 wt% | 9.4 | 100°C | 0.1 MPa | 40 min |
| Example 1-4 | Quartz | 10 wt% | 9.4 | 250°C | 4 MPa | 1 min |
| Example 1-5 | Quartz | 10 wt% | 9.4 | 200°C | 1.6 MPa | 240 min |
| Example 1-6 | Quartz | 20 wt% | 9.4 | 150°C | 0.5 MPa | 10 min |
| Example 1-7 | Quartz | 30 wt% | 9.4 | 150°C | 0.5 MPa | 10 min |
| Example 1-8 | Quartz | 10 wt% | 9.4 | 150°C | 0.5 MPa | 40 min |
| Example 1-9 | Quartz | 10 wt% | 9.4 | 250°C | 4 MPa | 40 min |
| Example 1-10 | Quartz | 10 wt% | 9.4 | 250°C | 4 MPa | 10 min |
| Example 1-11 | Quartz | 10 wt% | 9.4 | 250°C | 4 MPa | 40 min |
| Example 1-12 | Quartz | 5 wt% | 9.4 | 250°C | 4 MPa | 40 min |
| Example 1-13 | Quartz | 5 wt% | 9.4 | 250°C | 4 MPa | 120 min |
| Example 2-1 | Quartz | 10 wt% | 1.4 | 150°C | 0.5 MPa | 10 min |
| Example 2-2 | Quartz | 10 wt% | 2.5 | 150°C | 0.5 MPa | 10 min |
| Example 2-3 | Quartz | 10 wt% | 6.4 | 150°C | 0.5 MPa | 10 min |
| Comparative Example 2 | Quartz | 10 wt% | 12 | 150°C | 0.5 MPa | 10 min |
| Example 3 (MgCl₂ added) | Quartz | 10 wt% | 9.4 | 150°C | 0.5 MPa | 10 min |
| Example 4-1 | Cristobalite | 10 wt% | 9.4 | 150°C | 0.5 MPa | 10 min |
| Example 4-2 | Tridymite | 10 wt% | 9.4 | 150°C | 0.5 MPa | 10 min |
| Example 4-3 | Chrysotile | 10 wt% | 9.4 | 250°C | 4 MPa | 10 min |
| Example 4-4 | Chrysotile | 10 wt% | 9.4 | 250°C | 4 MPa | 40 min |
| Example 4-5 | Chrysotile | 10 wt% | 9.4 | 250°C | 4 MPa | 120 min |
| Example 5-1 | Quartz | 10 wt% | 9.4 | 150°C | 0.5 MPa | 10 min |
| Example 5-2 | Quartz | 10 wt% | 9.4 | 150°C | 0.5 MPa | 10 min |
| Example 5-3 | Quartz | 10 wt% | 9.4 | 150°C | 0.5 MPa | 10 min |
| Example 5-4 | Quartz | 10 wt% | 9.4 | 150°C | 0.5 MPa | 40 min |

**[Table 3]**

| | Impurity contained | Content | |
|---|---|---|---|
| | | Before hydrothermal reaction treatment | After hydrothermal reaction treatment |
| Example 1-1 | Quartz | 0.11 wt% | 0.01 wt% |
| Example 1-2 | Quartz | 0.11 wt% | 0.01 wt% |
| Example 1-3 | Quartz | 0.11 wt% | 0.01 wt% |
| Example 1-4 | Quartz | 0.11 wt% | 0.01 wt% |
| Example 1-5 | Quartz | 0.11 wt% | below the limit of detection |
| Example 1-6 | Quartz | 0.11 wt% | 0.03 wt% |
| Example 1-7 | Quartz | 0.11 wt% | 0.05 wt% |
| Example 1-8 | Quartz | 0.11 wt% | below the limit of detection |
| Example 1-9 | Quartz | 0.11 wt% | below the limit of detection |
| Example 1-10 | Quartz | 1 wt% | 0.01 wt% |
| Example 1-11 | Quartz | 1 wt% | below the limit of detection |
| Example 1-12 | Quartz | 5 wt% | 0.02 wt% |
| Example 1-13 | Quartz | 5 wt% | below the limit of detection |
| Example 2-1 | Quartz | 0.11 wt% | 0.03 wt% |
| Example 2-2 | Quartz | 0.11 wt% | 0.03 wt% |
| Example 2-3 | Quartz | 0.11 wt% | 0.01 wt% |
| Comparative Example 2 | Quartz | 0.11 wt% | 0.11 wt% |
| Example 3 (MgCl₂ added) | Quartz | 1 wt% | 0.03 wt% |
| Example 4-1 | Cristobalite | 0.12 wt% | 0.01 wt% |
| Example 4-2 | Tridymite | 0.12 wt% | 0.01 wt% |
| Example 4-3 | Chrysotile | 0.15 wt% | 0.075 wt% |
| Example 4-4 | Chrysotile | 0.15 wt% | 0.03 wt% |
| Example 4-5 | Chrysotile | 0.15 wt% | below the limit of detection |
| Example 5-1 | Quartz | 0.12 wt% | 0.04 wt% |
| Example 5-2 | Quartz | 0.12 wt% | 0.04 wt% |
| Example 5-3 | Quartz | 0.12 wt% | 0.01 wt% |
| Example 5-4 | Quartz | 0.12 wt% | below the limit of detection |

### <Test Example 1> Hydrothermal reaction treatment of natural talc containing quartz

### {Hydrothermal reaction treatment of natural talc}

A test was performed using a batch-type test machine (apparatus name: shaking-type reactor heating and stirring apparatus, manufactured by AKICO Co., Ltd.). After adding and well mixing 0.45 g of natural talc (concentration: 10 wt%) having an average particle diameter of 5 µm and containing quartz as an impurity with 4 mL of H₂O, in a 5 mL Inconel container, hydrothermal reaction treatment was performed for 10 minutes under the conditions described in Table 2 using the batch-type test machine. After cooling, processed talc was obtained by washing well with water.

### {Evaluation}

When the processed talc after hydrothermal reaction treatment was touched with a finger, it had a smooth texture similar to natural talc before hydrothermal reaction treatment.

The average particle diameter of the processed talc after hydrothermal reaction treatment was measured. As a result, in all examples, the average particle diameter was 5 µm.

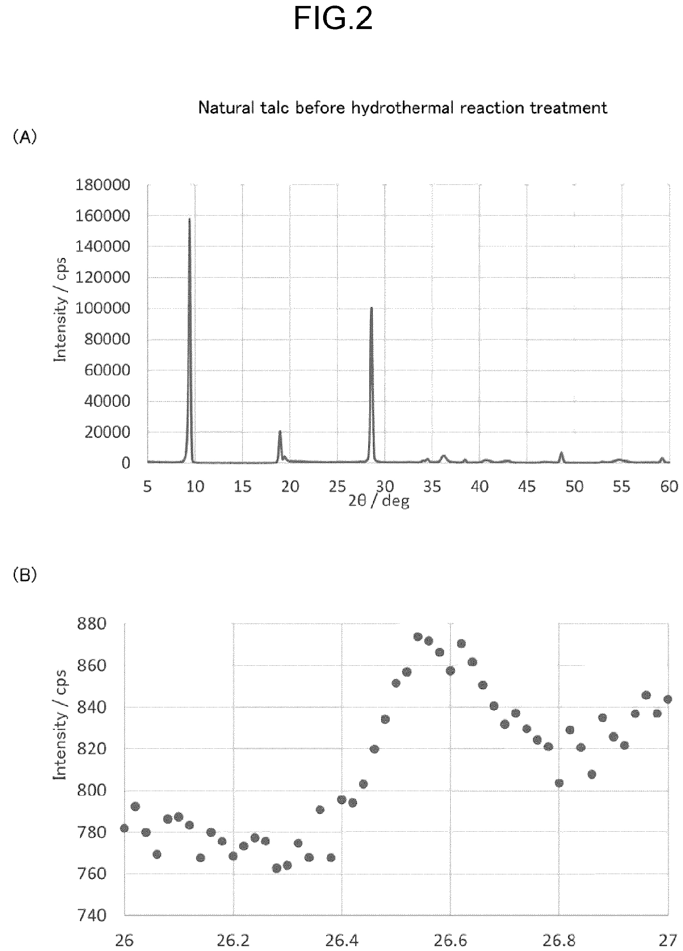

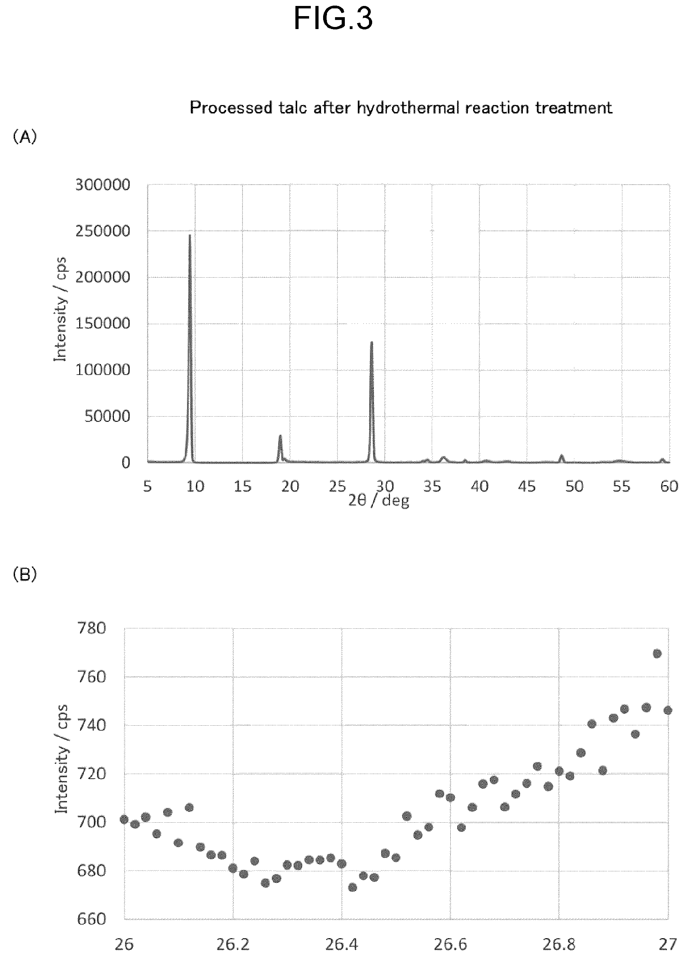

Further, powder X-ray diffraction was performed for each of the natural talc before hydrothermal reaction treatment and the processed talc after hydrothermal reaction treatment. FIG. 2 shows the result of the natural talc before hydrothermal reaction treatment, and FIG. 3 shows the result of the processed talc after hydrothermal reaction treatment according to Example 1-1. Further, Table 3 shows the change in impurity content between before and after hydrothermal reaction treatment in each example and comparative example. In both FIG. 2 and FIG. 3, diffraction peaks are present at diffraction angles (2θ) of 9.45°, 18.97°, and 28.62° in powder X-ray diffraction. This means that the substance after hydrothermal reaction treatment is talc.

On the other hand, at around 2θ=26.6°, before hydrothermal reaction treatment, a peak derived from quartz (crystalline silica) appears, and from a calibration curve obtained using a standard quartz sample, quartz is contained in the talc before hydrothermal reaction treatment at 0.11 wt%, whereas after hydrothermal reaction treatment, there is no alteration, and, on the other hand, the peak derived from quartz disappears. That is, in Example 1-1, it was confirmed that quartz can be sufficiently removed.

Similarly, in Examples 1-2 to 1-13, it was confirmed that quartz can be sufficiently removed from natural talc. In particular, in Examples 1-10 to 1-13, it was confirmed that quartz can be sufficiently removed from natural talc even when the natural talc has a relatively high impurity content. Further, in Examples 1-6 and 1-7, it was confirmed that quartz can be sufficiently removed from natural talc even if the natural talc concentration in hydrothermal reaction treatment is relatively high.

### <Test Example 2> Hydrothermal reaction treatment of natural talc containing quartz (pH dependence)

Except that the pH during hydrothermal treatment was adjusted to 1.4 (Example 2-1), 2.5 (Example 2-2), 6.4 (Example 2-3), and 12.0 (Comparative Example 2) by using nitric acid and sodium hydroxide, processed talc was obtained by the same method as in Example 1-1.

As a result of evaluation by XRD, in products obtained during hydrothermal treatment at pH 1.4, 2.5, and 6.4, decrease and disappearance of a peak derived from quartz could be confirmed. On the other hand, in a product obtained during hydrothermal treatment at pH 12.0, it was confirmed that a peak derived from quartz remained. Since the pH was 9.4 in Example 1-1, it was confirmed that quartz can be removed at a pH equal to or less than that, and conversely, if the pH is too high, quartz cannot be removed.

Note that, for products obtained at pH 1.4, 2.5, and 6.4, when the average particle diameter of primary particles was measured, all were 5 µm.

### <Test Example 3> Hydrothermal reaction treatment of natural talc containing quartz (addition of Mg ions)

Except that 0.06 g of natural talc (concentration: 1.5 wt%), 3 mg of MgCl₂ (concentration: 0.075 wt%), and 4 mL of H₂O were added to an Inconel container, and the hydrothermal reaction time was set to 240 minutes, processed talc was obtained by the same method as in Example 1-1.

As a result of evaluation by XRD, a decrease in a peak derived from quartz was confirmed. Even at a hydrothermal reaction temperature of 150°C and with natural talc having a relatively high impurity content equal to that in Example 1-10, a similar amount of quartz could be decreased.

### <Test Example 4> Hydrothermal reaction treatment of natural talc containing impurities different from quartz

### {Example 4-1} Hydrothermal reaction treatment of natural talc containing cristobalite

Apart from the fact that natural talc containing 0.12 wt% of cristobalite, which is a kind of crystalline silica, was used as the raw material, processed talc was obtained by the same method as in Example 1-1.

As a result of evaluation by XRD, since a peak at a diffraction angle (2θ) of 22.0° derived from cristobalite disappeared, it was confirmed that the cristobalite content can be reduced to 0.1 wt% or less by hydrothermal reaction treatment.

### {Example 4-2} Hydrothermal reaction treatment of natural talc containing tridymite

Except that natural talc containing 0.12 wt% of tridymite, which is a kind of crystalline silica, was used as the raw material, processed talc was obtained by the same method as in Example 1-1.

As a result of evaluation by XRD, since peaks at diffraction angles (2θ) of 20.5° and 21.6° derived from tridymite disappeared, it was confirmed that the tridymite content can be reduced to 0.1 wt% or less by hydrothermal treatment.

### {Examples 4-3 to 4-5} Hydrothermal reaction treatment of natural talc containing chrysotile

Processed talc was obtained by the same method as in Example 1-1 except that natural talc containing 0.15 wt% of chrysotile was hydrothermally treated at 250°C for 10 minutes, 40 minutes, and 120 minutes.

As a result of evaluation by XRD, the chrysotile content was 0.075 wt% (treatment time: 10 minutes), 0.03 wt% (treatment time: 40 minutes), and not more than the detection limit (treatment time: 120 minutes), and it was confirmed that the chrysotile content can be reduced to 0.1 wt% or less by hydrothermal reaction treatment.

In general, chrysotile is a stable substance, but under hydrothermal conditions, it tends to dissolve first from the tip of a needle-like structure of minerals, which occurs more easily than for ordinary minerals. However, what is important is the ratio of its dissolution rate and its dissolution amount to the dissolution amount of talc, and the amount of them remaining as a result, because even if it dissolves, the dissolution of talc may also occur. In consideration of the dissolution rate and dissolution amount of a large amount of silicate mineral, even if the same amount is dissolved, it becomes possible to ultimately leave only magnesium silicate. This result shows that a sufficient dissolution removal effect of chrysotile is observed, and shows that removal of an asbestos component from talc is possible.

### <Test Example 5> Hydrothermal reaction treatment of silicate minerals different from natural talc

### {Example 5-1} Hydrothermal reaction treatment of aluminum magnesium silicate containing quartz

Except that aluminum magnesium silicate containing 0.12 wt% of quartz was used as the raw material, a processed mineral was obtained by the same method as in Example 1-1.

As a result of evaluation by XRD, since a diffraction peak derived from quartz decreased, it was confirmed that the quartz content can be reduced to 0.04 wt% by hydrothermal treatment.

### {Example 5-2} Hydrothermal reaction treatment of calcium silicate containing quartz

Except that calcium silicate containing 0.12 wt% of quartz was used as the raw material, a processed mineral was obtained by the same method as in Example 1-1.

As a result of evaluation by XRD, since a diffraction peak derived from quartz decreased, it was confirmed that the quartz content can be reduced to 0.04 wt% by hydrothermal treatment.

### {Examples 5-3 and 5-4} Hydrothermal reaction treatment of magnesium silicate containing quartz

Except that magnesium silicate containing 0.12 wt% of quartz was used as the raw material, a processed mineral was obtained by the same method as in Example 1-1 (treatment times were 10 minutes and 40 minutes).

As a result of evaluation by XRD, since a diffraction peak derived from quartz disappeared, it was confirmed that the quartz content can be reduced to 0.01 wt% (treatment time: 10 minutes) and not more than the detection limit (treatment time: 40 minutes) by hydrothermal treatment.

### <Test Example 6> Relationship between hydrothermal treatment temperature and amount of OH groups and wettability on talc surface

**[Table 4]**

| Sample and hydrothermal reaction treatment conditions in each example and comparative example | | | | | | |
|---|---|---|---|---|---|---|
| | Sample conditions | | | Hydrothermal reaction treatment conditions | | |
| | Impurity | Talc concentration | pH | Temperature | Pressure | Treatment time |
| Example 6-1 | Quartz | 10 wt% | 9.4 | 150°C | 0.5 MPa | 10 min |
| Example 6-2 | Quartz | 10 wt% | 9.4 | 200°C | 0.5 MPa | 10 min |
| Comparative Example 6 | Quartz | 10 wt% | 9.4 | Untreated | | |

**[Table 5]**

| | Comparative Example 6 (before hydrothermal treatment) | Example 6-1 (150°C) | Example 6-2 (200°C) |
|---|---|---|---|
| Amount of NaOH aqueous solution used | 154 µl | 205 µl | 256 µl |
| Contact angle | ~140° | ~140° | ~130° |

Except that the conditions were those described in Table 4, a processed mineral was obtained by the same method as in Example 1-1.

### {Amount of OH groups on talc surface}

The amount of OH groups on the talc surface was evaluated using the Sears method. Specifically, it was performed by the following procedure.
(1) 0.1 g of the processed mineral was dispersed in 10 mL of water.
(2) After adding 2 g of NaCl, the pH was adjusted to 4 or less with dilute hydrochloric acid (0.12 M).
(3) Aqueous NaOH solution (0.01 M) was added to adjust the pH to 4, and then the amount required to reach pH 9 was examined.

The results are shown in Table 5. It was confirmed that, by hydrothermally treating natural talc, the amount of OH groups on the surface increased, and the amount of OH groups increased with increasing hydrothermal temperature.

### {Wettability}

For each of Example 6-2 and Comparative Example 6, talc was uniformly loaded on a sample holder for X-ray diffraction. Thereafter, one drop (10 µL) of water was dropped and the contact angle was observed.

The results are shown in Table 5. By hydrothermally treating natural talc, the contact angle became slightly smaller. This means that wettability has improved, that is, hydrophilicity had improved. It can thus be said that, by hydrothermally treating a silicate mineral, affinity with a polar solvent for cosmetics is improved and blendability is improved.

### <Test Example 7> Application to cosmetics

### {Test Example 7-1} Application to powder foundation

A powder foundation was prepared according to the formulation recipe shown in Table 6. In Example 7-1, hydrothermally treated talc obtained in Example 1-1 was used as talc. In Comparative Example 7-1, natural talc before hydrothermal treatment according to Example 1-1 was used as talc.

**[Table 6]**

| Component | % |
|---|---|
| Talc (*) | 27.90 |
| Mica | 30.00 |
| Silica | 7.00 |
| Boron nitride | 3.00 |
| Sodium dehydroacetate | 0.40 |
| Titanium oxide | 13.00 |
| Iron oxide | 3.00 |
| Ethylhexyl methoxycinnamate | 4.00 |
| Diisostearyl malate | 4.00 |
| Dimethicone | 4.00 |
| Squalane | 3.00 |
| Tocopherol | 0.20 |
| Sorbitan sesquiisostearate | 0.50 |
| Total | 100.00 |

| | |
|---|---|
| (*) Example 7-1: talc = hydrothermally treated talc obtained in Example 1-1 | |

### Comparative Example 7-1: talc = natural talc before hydrothermal treatment according to Example 1-1

For the obtained powder foundation, slurry state, hardness, drop strength, and amount taken were evaluated. As a result, it was confirmed that the powder foundation using hydrothermally treated talc has quality equivalent to that of the powder foundation using natural talc not hydrothermally treated.

### {Test Example 7-2} Application to loose powder

Loose powder was prepared according to the formulation recipe shown in Table 7. In Example 7-2, hydrothermally treated talc obtained in Test Example 7-1 was used as talc. In Comparative Example 7-2, natural talc before hydrothermal treatment according to Example 1-1 was used as talc.

**[Table 7]**

| Component | % |
|---|---|
| Talc (*) | 44.10 |
| Silica | 35.00 |
| Mica | 10.00 |
| Boron nitride | 8.00 |
| Sodium dehydroacetate | 0.50 |
| Iron oxide | 0.30 |
| Mica | 0.59 |
| Titanium oxide | 0.41 |
| Squalane | 1.00 |
| Tocopherol | 0.10 |
| Total | 100.00 |

| | |
|---|---|
| * Example 7-2: talc = hydrothermally treated talc obtained in Example 1-1 Comparative Example 7-2: talc = natural talc before hydrothermal treatment according to Example 1-1 | |

For the obtained loose powder, slurry state, hardness, drop strength, and amount taken were evaluated. As a result, it was confirmed that the loose powder using hydrothermally treated talc had quality equivalent to that of the loose powder using natural talc not hydrothermally treated.

### {Test Example 7-3} Application to eyebrow makeup

Two kinds of eyebrow makeups were prepared according to the formulation recipes shown in Table 8. In Examples 7-3-1 and 7-3-2, hydrothermally treated talc obtained in Example 1-1 was used as talc. In Comparative Examples 7-3-1 and 7-3-2, natural talc before hydrothermal treatment according to Example 1-1 was used as talc.

**[Table 8]**

| Component | Test Example 7-3-1 % | Test Example 7-3-2 % |
|---|---|---|
| Iron oxide | 50.00 | 34.15 |
| Talc (*) | 6.95 | 14.85 |
| Mica | 1.00 | 1.00 |
| Carnauba wax | 9.00 | 11.00 |
| Microcrystalline wax | 30.00 | 35.95 |
| Tri(caprylic/capric) glyceride | 1.00 | 1.00 |
| Squalane | 1.00 | 1.00 |
| Sorbitan sesquiisostearate | 1.00 | 1.00 |
| Tocopherol | 0.05 | 0.05 |
| Total | 100.00 | 100.00 |

| | | |
|---|---|---|
| * Example 7-3-1: talc = hydrothermally treated talc obtained in Example 1-1 Formulation recipe = that described in Test Example 7-3-1 Example 7-3-2: talc = hydrothermally treated talc obtained in Example 1-1 Formulation recipe = that described in Test Example 7-3-2 Comparative Example 7-3-1: talc = natural talc before hydrothermal treatment according to Example 1-1 Formulation recipe = that described in Test Example 7-3-1 Comparative Example 7-3-2: talc = natural talc before hydrothermal treatment according to Example 1-1 Formulation recipe = that described in Test Example 7-3-2 | | |

For the obtained eyebrow makeups, slurry state and drop strength were evaluated. As a result, it was confirmed that the eyebrow makeups using hydrothermally treated talc had quality equivalent to that of the eyebrow makeups using natural talc not hydrothermally treated.

### <Test Example 8> Application to skin-care cosmetics

### {Test Example 8-1} Application to body lotion for summer

A body lotion for summer was prepared according to the formulation recipe shown in Table 9. In Example 8-1, hydrothermally treated talc obtained in Example 1-1 was used as talc. In Comparative Example 8-1, natural talc before hydrothermal treatment according to Example 1-1 was used as talc.

**[Table 9]**

| Component | % |
|---|---|
| Tocopheryl acetate | 0.02 |
| Fragrance | 0.05 |
| l-Menthol | 0.10 |
| PEG-60 hydrogenated castor oil | 0.50 |
| Ethanol (99%) | 15.00 |
| 1,3-Butylene glycol | 5.00 |
| Nylon 12 | 1.00 |
| Talc (*) | 5.00 |
| Bentonite | 0.25 |
| Purified water | 73.08 |
| Total | 100.00 |

| | |
|---|---|
| * Example 8-1: talc = hydrothermally treated talc obtained in Example 1-1 Comparative Example 8-1: talc = natural talc before hydrothermal treatment according to Example 1-1 | |

For the obtained body lotion for summer, slurry state was evaluated. As a result, it was confirmed that the body lotion for summer using hydrothermally treated talc had quality equivalent to that of the body lotion for summer using natural talc not hydrothermally treated.

### {Test Example 8-2} Application to pore-concealing lotion

Pore-concealing lotion was prepared according to the formulation recipe shown in Table 10. In Example 8-2, hydrothermally treated talc obtained in Example 1-1 was used as talc. In Comparative Example 8-2, natural talc before hydrothermal treatment according to Example 1-1 was used as talc.

**[Table 10]**

| Component | % |
|---|---|
| Silica-treated (HDI/trimethylol hexyl lactone) crosspolymer | 5.00 |
| Talc (*) | 5.00 |
| Dimethicone elastomer aluminum-treated talc | 10.00 |
| Aluminum stearate-treated fine titanium dioxide | 1.00 |
| Organophilic bentonite | 3.50 |
| Sorbitan isostearate | 2.50 |
| PEG-10 dimethicone | 1.50 |
| Propylene glycol | 4.00 |
| Glycerin | 1.00 |
| Magnesium sulfate | 0.80 |
| Water-soluble collagen | 0.50 |
| Purified water | 35.00 |
| Decamethylcyclopentasiloxane | 30.20 |
| Total | 100.00 |

| | |
|---|---|
| * Example 8-2: talc = hydrothermally treated talc obtained in Example 1-1 Comparative Example 8-2: talc = natural talc before hydrothermal treatment according to Example 1-1 | |

For the obtained pore-concealing lotion, slurry state was evaluated. As a result, it was confirmed that the pore-concealing lotion using hydrothermally treated talc had quality equivalent to that of the pore-concealing lotion using natural talc not hydrothermally treated.

### {Test Example 8-3} Application to powder facial cleanser

A powder facial cleanser was prepared according to the formulation recipe shown in Table 11. In Example 8-3, hydrothermally treated talc obtained in Example 1-1 was used as talc. In Comparative Example 8-3, natural talc before hydrothermal treatment according to Example 1-1 was used as talc.

**[Table 11]**

| Component | % |
|---|---|
| Talc (*) | 55.00 |
| Potassium cocoyl glycinate | 15.00 |
| Crystalline cellulose | 6.00 |
| Sodium lauroyl glutamate | 6.00 |
| PVP | 5.00 |
| Trehalose | 5.00 |
| Mannitol | 5.00 |
| Potassium myristate | 3.00 |
| Total | 100.00 |

| | |
|---|---|
| * Example 8-3: talc = hydrothermally treated talc obtained in Example 1-1 Comparative Example 8-3: talc = natural talc before hydrothermal treatment according to Example 1-1 | |

For the obtained powder facial cleanser, slurry state was evaluated. As a result, it was confirmed that the powder facial cleanser using hydrothermally treated talc had quality equivalent to that of the powder facial cleanser using natural talc not hydrothermally treated.

### [Description of Reference Numerals]

- 1: Manufacturing apparatus
- 10: Extraction means
- 20: Cooling means

## Claims

1. A silicate mineral, wherein the contents of crystalline silica and asbestos are each 0.1 wt% or less.

2. The silicate mineral according to claim 1, containing a carbonate.

3. The silicate mineral powder according to claim 1, wherein the average primary particle diameter is 100 nm or more.

4. The silicate mineral powder according to claim 1, wherein the amount of an aqueous NaOH solution (0.01 M) required in the following method is 180 µL or more:
(1) Disperse 0.1 g of the silicate mineral powder in 10 mL of water.
(2) After adding 2 g of NaCl, adjust the pH to 4 or less with dilute hydrochloric acid (0.12 M).
(3) Add the aqueous NaOH solution (0.01 M) to adjust pH to 4, and then measure the amount of the aqueous NaOH solution (0.01 M) required to shift the pH from 4 to 9.

5. A cosmetic comprising the silicate mineral according to any one of claims 1 to 4.

6. A sanitary product comprising the silicate mineral according to any one of claims 1 to 4.

7. A pharmaceutical comprising the silicate mineral according to any one of claims 1 to 4.

8. A food comprising the silicate mineral according to any one of claims 1 to 4.

9. A method for producing a silicate mineral, comprising a step of subjecting a silicate mineral derived from a natural mineral to warm-water or hot-water treatment, or hydrothermal reaction treatment, at pH 9.4 or less.

10. The method according to claim 9, wherein the warm-water or hot-water treatment, or the hydrothermal reaction treatment is performed in the presence of carbonic acid or CO₂.

11. The method according to claim 9, wherein the warm-water or hot-water treatment, or the hydrothermal reaction treatment is performed in the presence of Mg ions.

12. The method according to claim 9, wherein the temperature in the warm-water or hot-water treatment, or the hydrothermal reaction treatment is from 70°C to 370°C, and the pressure is not less than the saturated vapor pressure of water.

13. The method according to claim 9, wherein the time of the warm-water or hot-water treatment, or the hydrothermal reaction treatment is 1 minute or more.

14. The method according to claim 9, wherein the warm-water or hot-water treatment, or the hydrothermal reaction treatment is performed using a batch apparatus or a semi-batch apparatus.

15. The method according to claim 14, wherein the reaction apparatus is a semi-batch apparatus,
and the amount of an aqueous solvent supplied to the semi-batch apparatus is 0.1 times or more of a theoretical amount required to saturate and dissolve crystalline silica contained in the silicate mineral as a raw material in a reaction solution containing coexisting ions in a reaction field.

16. The method according to claim 14, wherein the reaction apparatus is the semi-batch apparatus,
and the amount of crystalline silica contained in the silicate mineral as a raw material introduced into the semi-batch apparatus is 10 times or less of a theoretical amount required to saturate and dissolve the crystalline silica in a reaction solution containing coexisting ions in a reaction field.

17. The method according to claim 9, wherein, using a flow-through apparatus, the silicate mineral is supplied in a suspended state in water, and the warm-water or hot-water treatment, or the hydrothermal reaction treatment, is performed.

18. The method according to claim 17, wherein a concentration of a silicate-mineral aqueous slurry supplied to the flow-through apparatus is 0.1 times or more of the theoretical amount required to saturate and dissolve crystalline silica contained in the silicate mineral as a raw material in a reaction solution containing coexisting ions in a reaction field.
